(19) **Europäisches Patentamt**
**European Patent Office**
**Office européen des brevets**

(11) **EP 2 922 581 B1**

(12) **EUROPEAN PATENT SPECIFICATION**

(45) Date of publication and mention
of the grant of the patent:
**15.03.2017 Bulletin 2017/11**

(51) Int Cl.:
***A61L 26/00*** *(2006.01)*

(21) Application number: **12791441.4**

(86) International application number:
**PCT/EP2012/071138**

(22) Date of filing: **25.10.2012**

(87) International publication number:
**WO 2014/063735 (01.05.2014 Gazette 2014/18)**

(54) **MUCOADHESIVE COMPOSITIONS COMPRISING HYALURONIC ACID AND CHITOSAN FOR TOPICAL APPLICATION**

MUKOADHÄSIVE ZUSAMMENSETZUNGEN MIT HYALURONSÄURE UND CHITOSAN ZUR TOPISCHEN ANWENDUNG

COMPOSITIONS MUCOADHÉSIVES COMPRENANT DE L'ACIDE HYALURONIQUE ET DU CHITOSANE DESTINÉES À UNE APPLICATION TOPIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**30.09.2015 Bulletin 2015/40**

(73) Proprietor: **MDT INT'L S.A.**
**1207 Geneva (CH)**

(72) Inventors:
• **BETTINI, Ruggero**
**43035 Felino (IT)**
• **BIANCHERA, Annalisa**
**43125 Parma (IT)**
• **CORUZZI, Massimo**
**43124 Vigatto (IT)**

• **ELVIRI, Lisa**
**43123 Parma (IT)**

(74) Representative: **Gervasi, Gemma et al**
**Notarbartolo & Gervasi S.p.A.**
**Corso di Porta Vittoria 9**
**20122 Milano (IT)**

(56) References cited:
**US-A1- 2006 280 797**

• **CHENITE A ET AL: "Novel injectable neutral solutions of chitosan form biodegradable gels in situ", BIOMATERIALS, ELSEVIER SCIENCE PUBLISHERS BV., BARKING, GB, vol. 21, no. 21, 1 November 2000 (2000-11-01), pages 2155-2161, XP004216030, ISSN: 0142-9612, DOI: 10.1016/S0142-9612(00)00116-2**

EP 2 922 581 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the invention

The present invention relates to aqueous compositions of hyaluronic acid combined with chitosan and glycerophosphate having thermo-gelling and mucoadhesive properties and use thereof for mucosal wound re-epithelisation and repair by topical administration.

### Background of the invention

The hyaluronic acid (hereinafter referred to as HA) is a well-known high molecular weight biological polysaccharide having a polyanionic nature and belonging to the class of non-sulphate glycosaminoglycans. It is present to a great extent in all connective tissues of vertebrates, in joint synovial fluid and cartilage, where it is a major component, and eye endobulbar fluids.

In nature it can reach a molecular weight higher than 10,000 kDa, but, as known, when extracted and manipulate the hyaluronic acid is always a fraction commonly identified by an average molecular weight, being formed by different polymers having molecular weight comprised in a range of molecular weights, namely it is characterized by an index of polydispersity.

It is widely employed for many medical applications, including administration to eyes, joint, mucosa and skin for treating a variety of pathological conditions.

At present, a lot of fractions of this biopolymer extracted from animal sources or bacterial broth are in use for long in pathological conditions such as wound healing, arthrosic/arthritic diseases and ocular surgery, these uses being essentially based on its biological role and on the non-newtonian physical properties (e.g. viscosity and/or its structural viscoelasticity) of its aqueous solutions. Both viscosity and/or viscoelastic properties are mainly dependent on molecular weight range of HA fractions, polymer concentration, but other technical features, such as degree of chain cross-linkage with chemical agents, can contribute to increase the rheological performance as well as to the claimed molecular weight.

In fact, the rheological properties of HA are influenced or resulting from the combination of the different technical features of the sample considered.

Notwithstanding, it is common knowledge and practice to refer to a high molecular weight HA fractions when a high viscosity and/or viscoelastic properties are requested, in particular, for instance, in knee joint inflammatory conditions or ocular surgery, where hyaluronic acid is employed for supplementation purpose of the synovial or endobulbar fluids for its viscosity/viscoelastic properties.

As far as the wound healing property is concerned, this is mainly attribute to hyaluronic acid fractions having essentially low molecular weights and a fraction of this polysaccharide having a molecular weight from 50 to 100 kDa has been claimed to exhibit a low viscosity in aqueous solutions but to have the capability to promote wound tissue repair (US 5,925,626).

The HA fraction claimed, as other low molecular weight HA fractions, do not show any mucoadhesive property, but this property can be particularly useful for the topical application thereof.

In fact, the main need for wound healing, and in particular for the treatment of internal mucosal tissues of body inner cavities, where a wound dressing is not applicable, is to make available at the site to be treated an appropriate amount of hyaluronic acid and for a sufficient time to exploit its biological activity.

WO2009/024677 discloses the use of hyaluronic acid, preferably associated to vitamins, for the preparation of compositions addressed to improve by topical administration the protection of mucosal tissue of the higher and lower respiratory airways, of intestinal tract and of the eye. The compositions usable for the intestinal tract mucosa treatment are orally administered in gastro-resistant pharmaceutical forms.

However, for improving the retention time at the site of application, a feature particularly significant is the bioadhesive and mucoadhesive property of the compositions used.

For example, for treating skin and mucosa epithelia highly bioadhesive and mucoadhesive compositions of biopolymers, such as alginic acid, hyaluronic acid and dermatan sulfate, associated with synthetic polymers, such as polyvinyl alcohol and polycarbophyl, are disclosed (WO96/03973). These compositions are claimed to be useful for the treatment of skin and mucosal tissues dryness and dehydration as well as carriers for percutaneous absorption of active substances.

A biopolymer, which is currently receiving a great deal of attention for skin and mucosal topical applications for its capacity to bind high quantity of water and to form hydrogels is chitosan. Chitosan is a deacetylated derivative of chitin consisting of glucosamine and N-acetyl-glucosamine units; it is a biocompatible, biodegradable, non-toxic, cationic polymer, insoluble in water, but soluble in diluted aqueous acids up to pH 6.2. It is widely used in many applications, but its poor solubility in neutral solutions constitutes a major obstacle to its use in physiological contexts. In order to overcome this drawback, chitosan backbone was variously chemically modified at the end to obtain a covalently grafted or a crosslinked chitosan and to improve in this manner its water solubility and gelling properties. However, such chemical modifications often require the use of toxic reagents which partially hamper the translation of such products to biomedical applications.

A possible means to avoid the use of chemical agents is to prepare physical chitosan hydrogels exploiting its cationic nature in forming polyelectrolyte complexes (PEC) by ionic interactions with polyanions consisting of both synthetic polymers and biopolymers, such as for example polyvinyl alcohol and anionic polysaccharides, including hyaluronic acid, respectively (Berger J. et al., Eur. J. Pharmaceutics and Biopharmaceutics, 2004, 57, 35-52).

However, the formation of stable hydrogels, homogenously dispersed, containing chitosan and HA in solution is challenging due to the fact that the addition of HA to the chitosan solution constitutes a perturbing element, potentially conflicting. In fact, the basic charges of chitosan lead to anionic interactions with the anionic charges of HA and consequently affords precipitation of a HA-chitosan polyelectrolyte complex with phase separation. This occurrence is so common that it is broadly exploited and reported as a strategy for the production of micro or nanoparticles (some examples are listed in Berger J. et al., previously cited). Another peculiar characteristic of chitosan is its capability to form thermosensitive gels with polyols bearing a single phosphate anionic head, and in particular with glycerophosphate.

US 6,344,488 discloses a temperature-controlled and pH-dependent formation of ionic polysaccharides gels consisting in a system of chitosan/organo-phosphate aqueous solutions displaying a sol/gel transition within temperatures comprised from 30 to 60°C and pH comprised from 6.5 to 7.4. The gels obtained at pH higher than 6.9 are thermoirreversible, whilst the gels obtained at pH lower than 6.9 are thermoreversible. Furthermore, in the patent is clearly indicated that chitosan has a molecular weight comprised from 10 to 2,000 kDa, but the patent is silent on the other feature of chitosan, namely the degree of deacetylation. The preferred organo-phosphate is glycerophosphate. US 6,344,488 also discloses the method of preparation of hydrogels and describes in details their behavior after heating but does not include the case of the addition of further active substances or other polysaccharides such as hyaluronic acid.

Afterwards, the mechanism of formation of thermo-sensitive gels by the association of chitosan and glycerophosphate salts is reported by the same authors (Chenite A. et al., Biomaterials, 2000, 21, 2155 - 2161; Chenite A. et al., Carbohydr. Polym., 2001,46,39 - 47). According to the data shown, the presence of glycerophosphate allows a higher hydration of chitosan at low temperatures, thus promoting the stability of the solution at pH near neutrality. It is worthwhile to note that in the papers in reference the chemical features of chitosan (namely degree of deacetylation and molecular weight) are considered. The results shown indicate that the molecular weight has no significant effect on the gelation temperature, whereas the degree of deacetylation seems to be critical for the temperatures at which the sol/gel transition occurs. In particular, the temperature of incipient gelation increases as the degree of deacetylation decreases. For chitosan having a degree of deacetylation of 70, 81 and 91% the gelation temperatures reported are 66°C, 57°C and 37°C respectively. The deacetylation degree is also critical for hydrogels stability (Ruel-Gariépy E. et al., Int. J. Pharmaceutics, 2000, 203, 89-98).

US2006/0280797 describes a polymer matrix comprising a solution of at least one inverse thermal gelling polymer, e.g. a chitosan and ß-glycerophosphate solution, and at least one anionic gelling polymer in powdered form, e.g. hyaluronic acid, wherein the polymer matrix is a solid gel that is injectable due to shear thinning properties of the anionic gelling polymer and returns to its original viscosity after shearing.Data concerning the production of thermosensitive hydrogel containing chitosan and hyaluronic acid are rare.

Fang et al. (Eur. J. Pharmaceutics and Biopharmaceutics, 2008, 68, 626-636) and Chen et al. (Polymer, 2009, 50, 107-116) report about thermosensitive hydrogels composed of chitosan and hyaluronic acid but in both cases a chemical modification of chitosan, HA or both is required to overcome the incompatibilities between these molecules.

As previously mentioned, in fact, the preparation of homogeneously dispersed hydrogels combining chitosan and a polyanionic polysaccharide such as hyaluronic acid is not a trivial problem.

## Summary of the invention

The purpose of the present invention is to provide compositions comprising hyaluronic acid in an aqueous solution for treating mucosal lesions by topical administration and particularly for treating mucosa of body cavities, such as oral, nasal, gastrointestinal, colorectal and vaginal cavities either of traumatic origin, iatrogenic origin or disorder-related origin. The administration of HA is addressed to the relief of mucosal lesions, with a particular interest in the lesions derived from chemotherapy and/or radiation therapy used to treat cancer.

The main need for the wound healing of tissues, such as the mucosal tissues, is to make available at the site an appropriate amount of hyaluronic acid with a contact time with the lesion sufficient for providing relief of the tissue and then to trigger the healing processes.

It is a further purpose of the present invention to provide compositions comprising hyaluronic acid in an aqueous solution capable to form hydrogel, preferably *in situ* after topical application.

For the aforementioned purposes, an ideal composition should possess the characteristics of biocompatibility, safety and mucoadhesiveness, in order to guarantee a good persistence at the site of administration. The capability to be thermosensitive in forming hydrogels is also a relevant feature in view of the application pursued.

Compositions where the hyaluronic acid is combined with chitosan and sodium glycerophosphate are envisaged as potentially useful for the delivery of hyaluronic acid at the site to be treated.

Therefore, in a first aspect the object of the invention are compositions comprising hyaluronic acid and salts thereof in admixture with chitosan and salts thereof and a salt of glycerophosphate in an aqueous solution, wherein the chitosan has a deacetylation degree in the range from 60 to 80%, and wherein said compositions have a sol-gel transition property at 35-38°C so forming at said temperatures a hydrogel. Moreover, these compositions are further endowed with mucoadhesive properties. The compositions comprising hyaluronic acid and salts thereof in admixture with chitosan and a salt of glycerophosphate optionally further comprise an inorganic or organic acid salt.

The compositions of the invention are intended for use in treating mucosal wounds, either of traumatic, iatrogenic or disorder-related origin, of the body oral, nasal, gastrointestinal, vaginal, colorectal cavities by topical administration. The preferred use is for the relief (re-epithelisation and repair) of mucosal lesions derived from chemotherapy and/or radiation therapy and in particular for colorectal mucosal lesions derived from radiation therapy used to treat colorectal cancer.

These purposes and others, that will become clear from the following detailed description, are achieved by the compositions comprising hyaluronic acid combined with chitosan and glycerophosphate in aqueous solution object of the invention.

Other characteristics and advantages of the present invention will be described in the following detailed description of preferred, but not exclusive, embodiments of the present invention.

## Brief description of the figures

The description is given with reference to the enclosed figures, which are provided purely for indicative purpose and then are non-limiting.

Figure 1 shows the percentage of bound water in the compositions prepared according to examples 1-3 and 7 versus the ratio between the concentrations of chitosan and of HA.

Figure 2 shows the percentage of HA released as a function of time from composition of example 1; the bars represent the standard deviation (n=3).

Figure 3 shows the percentage of HA released as a function of time from Carbopol-based composition corresponding to composition 1; the bars represent the standard deviation (n=3).

Figure 4 shows the percentage of HA released as a function of time from chitosan-based composition of example 2; the bars represent the standard deviation (n=3).

Figure 5 shows the percentage of HA released as a function of time from Carbopol-based composition corresponding to composition of example 2; the bars represent the standard deviation (n=3).

## Detailed description of the invention

For the purpose of the present invention the following terms and expressions used in the overall description are defined below.

The term "hyaluronic acid" is herein intended to mean a fraction of hyaluronic acid extracted from animal sources or obtained by microbial fermentation having a molecular weights ranging from 7-250 kDa (7,000-250,000). The intrinsic viscosity of this fraction determined according to Ph. Eur. 7.0, 2011 page 2928, is lower than 0.65 $m^3$/kg.

For the purpose of the invention the preferred hyaluronic acid is a fraction having a molecular weight ranging from 30 to 250 kDa and more preferably from 30 and 100 kDa. The hyaluronic acid is preferably in form of salt with an alkaline metal and more preferably is in in form of sodium salt, namely sodium hyaluronate.

The term "chitosan" is herein intended to define a polysaccharide derived from deacetylation of chitin, having a degree of deacetylation (herein referred to as DD) ranging from 60 to 80% obtained from natural sources, such as crustaceous shell or from the cell wall of fungi. The molecular weight of said chitosan is comprised in the range from 50 to 300 kDa and preferably from 80 and 200 kDa.

For the purpose of the invention the preferred chitosan has a degree of deacetylation of around 70-80%. The 75% deacetylated chitosan is the most preferred and is a chitosan a viscosity between 30 and 70 mPas at 1% concentration(w/v) in 1 % acetic acid at 20 °C.

Furthermore, a chitosan having the referred deacetylation degree can be in form of salt and can selected from hydrochloride sulfate, acetate, lactate, malate, succinate, ascorbate, fumarate, adipate chitosan salts.

The term "glycerophosphate" is herein intended to mean an alkaline or an earth-alkaline metal, preferably sodium, salt of D or L α-glycerophosphoric acid, β-glycerophosphoric acid and mixture thereof. It can be prepared from natural sources, such as for example by hydrolysis of lecithin or by phosphorylation of glycerol. For the purpose of the invention the preferred glycerophosphate is a sodium salt of β-glycerophosphoric acid and is according to Ph. Eur. 7.0, namely as hydrate crystalline material with 4 to 6 molecules of water of crystallization. The sodium pentahydrate glycerophosphate is the most preferred.

The term "polysaccharide(s) solution(s)" is/are herein intended to mean aqueous clear solutions of the related polysaccharide not gelled and stable at temperatures ranging from 0°C to room temperature (e.g. 15-25°C).

The terms "sol/gel transition temperature" and "gelation temperature" are equivalent and are herein intended to mean any temperature ranging from 34°C to about 40 °C, preferably 35-38°C, at which an aqueous clear solution comprising hyaluronic acid, chitosan and glycerophosphate turns a hydrogel homogeneously dispersed without any formation of precipitates and phase separation.

In order to fulfill the purpose of the invention, that is to provide mucoadhesive hydrogel compositions comprising hyaluronic acid for topical application thereof on the inner mucosal tissues of a body cavity, an aqueous composition based on chitosan and sodium glycerophosphate solutions was envisaged as a potential useful system to be exploited for the development of a suitable medical device. As mentioned in the State-of the Art, it is known that such system possesses the distinctive feature of being able to form thermo-sensitive hydrogels, consistently increasing its viscosity upon heating, whereas the mucoadhesive properties or the addition of other components were not investigated.

Nevertheless, it is expected that the addition to this system of a polyanion, such as hyaluronic acid, gives rise to the technical problems previously mentioned due to the mutual incompatibility in aqueous solution between the two polysaccharides taken as a such.

Therefore, for compositions comprising hyaluronic acid and salts thereof combined with chitosan and salts thereof and a salt of glycerophosphate in an aqueous solution endowed with a sol-gel transition at 35-38 °C and mucoadhesive properties, this incompatibility has to be solved in order to obtain stable hydrogels and to avoid the formation of polyelectrolyte complexes by ionic interactions between chitosan and hyaluronic acid with phase separation.

The achievement of this purpose relies on a series of technical features: the characteristics of polysaccharides used, the ratio between them and the pH.

The HA has been selected from those fractions presenting a low molecular weight for its known properties on wound healing and but even for technological reason. In fact, it is widely reported in literature that naturally occurring fragments derived from the degradation of HA actively participate in tissue remodeling and regeneration, thus playing a crucial role in wound healing, and at the same time it is known that the size of the polymer can deeply affect its behavior in the composition, as the number of interactions between the two polymeric chains is strictly dependent on their molecular size. Then, the HA for the compositions according to the invention has a molecular weight from 7 to 250 kDa and preferably from 30 to 250 kDa and more preferably from 30 to 100 kDA.

As for chitosan, contrary to what disclosed in the state of the art for the compositions of the present invention the chitosan best performing with reference to gelation temperature in the physiological range around 35-38 °C is the chitosan having a degree of deacetylation of 75%, while with a chitosan having a DD of 95% the hydrogels obtained shows at temperature between 0 and 40 °C formation of coacervates with sudden phase separation. Therefore the chitosan or salts thereof for the compositions according to the invention has a degree of DD from 60-80% and preferably from 70-80% and more preferably has a degree of DD of 75%. The most critical feature for the compositions comprising hyaluronic acid and salts thereof combined with chitosan and salts thereof and sodium glycerophosphate in an aqueous solution are the ratios between chitosan and HA. Considering chitosan, these ratios can be comprised from 1:0.6 (HA:Chitosan) and to 1:45 (HA:Chitosan). Preferably, the ratios between HA and chitosan are: 1:10 (HA:chitosan); 1:6 (HA:chitosan); 1:3 (HA:chitosan) and 1:1.8 (HA:chitosan).

For the thermogelling capability the ratios between chitosan and glycerophosphate can be comprised from 1:0.8 to 1:15 (chitosan: glycerophosphate). The preferred ratios are from 1:1.8 to 1:7.0 (chitosan: glycerophosphate).

Therefore, in one embodiment for the compositions of the invention comprising hyaluronic acid and salts thereof combined with chitosan and salts thereof and a salt of glycerophosphate in an aqueous solution of the present invention, the contents of the components are in the following ranges in % w/v:

- hyaluronic acid from 0.1 to 1.5;
- chitosan from 1.0 to 4.5; and
- glycerophosphate from 4.0 to 15 as anhydrous substance

and the pH is comprised from 6.9 to 7.1.

In a preferred embodiment the compositions of the invention comprise:

- hyaluronic acid from 0.3 to 1.0;
- chitosan from 1.8 to 3.0; and
- glycerophosphate from 5.6 to 12.7 as anhydrous substance

and the pH is comprised from 6.9 to 7.1,

in this case, when the glycerophosphate is according to Ph. Eur. 7.0 and is as pentahydrate substance, the hereinabove reported ranges are in % w/v from 8 to 18%. Furthermore, for the purposes of the invention the glycerophosphate pentahydrate is preferably 8% by w/v.

Therefore, the preferred compositions of the invention are: hyaluronic acid 0.3%, chitosan 3.0% and sodium glycerophosphate pentahydrate 8.0% by w/v; hyaluronic acid 0.3%, chitosan 1.8% and sodium glycerophosphate pentahydrate 8.0% by w/v; hyaluronic acid 1.0%, chitosan 3.0% and sodium glycerophosphate pentahydrate 8.0% by w/v and hyaluronic acid 1.0%, chitosan 1.8% and sodium glycerophosphate pentahydrate 8.0% by w/v. The latter composition is the most preferred.

When the chitosan used is not in form of salts, the compositions can further comprise an inorganic or organic acid in a range from 0.6 to 0.9% w/v in order to solubilize the chitosan in water and to provide a pH nearly in the physiological range, namely 6.9 and 7.1. Said inorganic or organic acids are selected from the group consisting of hydrochloride, sulfuric, acetic acid, lactic acid ascorbic, malic, succinic, adipic or fumaric, preferably the acid is lactic acid.

Among compositions capable to afford thick gels upon heating at 37°C, two are the most preferred: composition comprising 0.3% HA, 3% chitosan, 8% sodium glycerophosphate pentahydrate and 0.9% lactic acid and composition comprising 1.0% HA, 1.8% chitosan, 8.0% sodium glycerophosphate pentahydrate and 0.6% lactic acid.

In one embodiment the compositions can be prepared by mixing water solutions of the single components in adequate proportions to obtain the concentration pursued at temperatures ranging from 0 to 25 °C. The water solution of chitosan is added with an inorganic or organic acid selected from hydrochloric, sulfuric, acetic, lactic, ascorbic, malic, succinic, adipic or fumaric acid, and preferably is added with lactic acid, when chitosan is not in form of salt. The temperatures of preparation do not affect the gelation upon heating at 37°C, but strongly influence the appearance of compositions in terms of clearness and homogeneity. Actually, compositions prepared at temperatures greater than 10 °C can present an opalescence/turbidity with signs of phase separation. For this reason the temperatures from 0 to 4 °C are the starting temperatures for the preparation of the compositions to be preferred.

Water plays a very critical role on the behavior of these compositions, in particular the higher is the amount of bound water, the better are the gel characteristics: the quantification of free water in formulation accounted for about 90% of total water, significantly affecting the stability of compositions. None of the compositions, in any of the storing conditions tested, namely at 4 °C or at 25 °C or at 40 °C, showed significant signs of thermoreversibility, except for a slight decrease in viscosity for compositions having higher content of HA freshly prepared or after 4 °C storage.

In a further embodiment the compositions of the invention can be obtained by preparing two separate starting water solutions, one containing an acidic water solution of chitosan or a water solution of a chitosan salt and the other a water solution of sodium glycerophosphate and HA, to be mixed at the moment of administration. The starting solutions can be two times concentrated with respect to the final concentration in the desired composition and can be mixed at room temperature through a suitable device, for example an automatic injector by linking the two syringes to a Y-connection. In these conditions the resulting solution still retains the ability to become a thick gel in less than 15 minutes when heated at 37 °C. This is quite interesting especially considering that all the steps can be performed at room temperature, thus eliminating the need of cooling down solutions before mixing, and the hydrogels can be formed *in situ* at the moment of the administration to the subject in a need of the treatment.

Hereinafter are briefly reported the results obtained with some embodiments of the compositions of the invention and disclosed in detail in the examples.

As a first step, water content in the compositions prepared as examples was quantified by Thermo Gravimetric Analysis (TGA) resulting in good agreement with nominal amount (data not shown). Water plays a very critical role on the behavior of compositions, in particular the higher is the amount of bound water the better are the gel characteristics, as its interactions with polymeric chains of chitosan determine the ability of the composition to become a gel as well as its stability. In order to quantify the amount of free water, which is freezable, Differential Scanning Calorimetry (DSC) experiments were performed on compositions having different content of chitosan and hyaluronic acid: the amount of free water resulted respectively in 87.2% (composition ratio chitosan:HA equal to 10:1) and 91.9% (composition ratio chitosan:HA equal to 1.8:1). In other words the amount of bound water was 12.8 for the composition ratio chitosan:HA equal to 10:1 and 8.1 for the composition ratio chitosan:HA equal to 1.8:1. This means that the amount of bound water increased concomitantly with the relative ratio between chitosan and hyaluronic acid, thus suggesting that the binding of water is mainly due to chitosan.

Compositions according to the present invention have been tested in an *ex-vivo* experiment for mucoadhesiveness. Pig mucosa was selected as a model and a wash-off test was performed versus a Carbopol®-based formulation taken as a golden standard. The results of the test revealed that the compositions tested had a higher mucoadhesiveness with respect to the corresponding Carbopol®-based composition.

Thus, distinctive features of the compositions of the invention are:

- the presence of components which are biologically safe and biocompatible;

- the coexistence in water solution of a basic compound, such as chitosan, and an acid one, such as hyaluronic acid, without chemical modifications of both chitosan and hyaluronic acid, thus totally avoiding any risk of toxicity derived from chemical or photo-initiated crosslinking;

- the ability to form hydrogels at 37°C;
- high mucoadhesiveness, significantly higher than the golden standard.

In conclusion, the compositions according to the present invention constitute an innovative medical delivery system for the mucosal administration of hyaluronic acid. They possess the distinctive characteristics of allowing the coexistence in solution of a basic compound such as chitosan and an acid one such as hyaluronic acid. Moreover, their ability to gelify at 37 °C accompanied by high mucoadhesiveness constitutes a good solution for administration of *in situ* formed hydrogels and for an efficacious and persistent localization of HA at the site of damage.

EXAMPLES

In the following examples are described the preparation of composition where hyaluronic acid, chitosan and glycerophosphate are combined in different amounts and the chitosan has a deacetylation degree of 75% (examples 1-8) or, as comparison, a deacetylation degree of 95% (examples A-F). Hereinafter are also described the characterization test performed on compositions according to the invention.

Example 1 *Preparation of a composition of hyaluronic acid 0.3%, chitosan 3.0% and sodium glycerophosphate pentahydrate 8.0% by w/v.*

A first aqueous solution of chitosan (4% w/v) was prepared by adding to a water dispersion of chitosan, having a degree of deacetylation of 75%, lactic acid up to a concentration of 1% w/v. Separately were prepared a 50% w/v aqueous solution of sodium glycerophosphate pentahydrate (NaGP) and a 10% w/v aqueous solution of sodium hyaluronate (HA). All the aqueous solutions were prepared at 0 °C. Then, the aqueous solutions obtained were stabilized in a cryostat at temperatures between 0 to 25°C.

To obtain the final concentrations of hyaluronic acid 0.3%, chitosan 3.0% and NaGP 8.0% by w/v, samples of known volumes of the 50% w/v aqueous solution of NaGP and the 10% w/v aqueous solution of sodium HA were added to a sample of known volume of the first aqueous solution of 4% chitosan (concentration of lactic acid = 0.9% w/v). The three aqueous solutions were mixed with vortex or magnetic stirring at room temperature, obtaining a homogeneous and clear solution with a final pH of 6.9.

Example 2 *Preparation of a compositions of hyaluronic acid 1.0%, chitosan 1.8% and sodium glycerophosphate pentahydrate 8.0% by w/v.*

A first aqueous solution of chitosan (4% w/v) was prepared by adding to a water dispersion of chitosan, having a degree of deacetylation of 75%, lactic acid up to a concentration of 1 % w/v. Separately were prepared a 50% w/v aqueous solution of sodium glycerophosphate pentahydrate (NaGP) and a 10% w/v aqueous solution of sodium hyaluronate (HA). All the aqueous solutions were prepared at 0 °C. The aqueous solutions obtained were, then, stabilized in a cryostat at temperatures between 0 to 25°C.

To obtain the final concentrations of three components (hyaluronic acid 1.0%, chitosan 1.8% and NaGP 8.0% by w/v),samples of known volumes of the 50% w/v aqueous solution of NaGP and the 10% w/v aqueous solution of HA were added to a sample of known volume of the first aqueous solution of chitosan 4% (concentration of lactic acid = 0.6% w/v). The mixture of the three component aqueous solutions were mixed with vortex or magnetic stirring at room temperature, obtaining a homogeneous and clear solution with a final pH of 6.9.

Example 3 *Preparation of a compositions of hyaluronic acid 1.0%, chitosan 3.0% and sodium glycerophosphate pentahydrate 8.0% by w/v.*

A first aqueous solution of chitosan (4% w/v) was prepared by adding to a water dispersion of chitosan, having a degree of deacetylation of 75%, lactic acid up to a concentration of 1% w/v. Separately were prepared a 50% w/v aqueous solution of sodium glycerophosphate pentahydrate (NaGP) and a 10% w/v aqueous solution of sodium hyaluronate (HA). All the aqueous solutions were prepared at 0 °C. Then, the aqueous solutions obtained were stabilized in a cryostat at temperatures between 0 to 25°C.

To obtain the final concentrations of three components (hyaluronic acid 1.0%, chitosan 3.0% and NaGP 8.0% by w/v), samples of known volumes of the 50% w/v aqueous solution of sodium NaGP and the 10% w/v aqueous solution of sodium HA were added to a sample of known volume of the first aqueous solution of chitosan 4% (concentration of lactic acid = 0.9% w/v). The mixture of the three component aqueous solutions were mixed with vortex or magnetic stirring at room temperature, obtaining a homogeneous and clear solution with a final pH of 6.9. Example 4 *Preparation of a compositions of hyaluronic acid 0.3%, chitosan 3.0% and sodium glycerophosphate pentahydrate 18% by w/v.*

A first aqueous solution of chitosan (4% w/v) was prepared by adding to a water dispersion of chitosan, having a degree of deacetylation of 75%, lactic acid up to a concentration of 1 % w/v. Separately were prepared a 50% w/v aqueous solution of sodium glycerophosphate (NaGP) and a 10% w/v aqueous solution of sodium hyaluronate (HA). All the aqueous solutions were prepared at 0 °C. Then, the aqueous solutions obtained were stabilized in a cryostat at temperatures between 0 to 25°C.

To obtain the final concentrations of three components (hyaluronic acid 0.3%, chitosan 3.0% and sodium NaGP 18% by w/v, samples of known volumes of the 50% w/v aqueous solution of NaGP and the 10% w/v aqueous solution of sodium HA were added to a sample of known volume of the first aqueous solution of chitosan 4% (concentration of lactic acid = 0.9% w/v). The mixture of the three component aqueous solutions were mixed with vortex or magnetic stirring at room temperature, obtaining a homogeneous and clear solution with a final pH of 7.0.

Example 5 *Preparation of a compositions of hyaluronic acid 1.0%, chitosan 1.8% and sodium glycerophosphate pentahydrate 18% by wlv.*

A first aqueous solution of chitosan (4% w/v) was prepared by adding to a water dispersion of chitosan, having a degree of deacetylation of 75%, lactic acid up to a concentration of 1% w/v. Separately were prepared a 50% w/v aqueous solution of sodium glycerophosphate pentahydrate (NaGP) and a 10% w/v aqueous solution of sodium hyaluronate (HA). All the aqueous solutions were prepared at 0 °C. Then, the aqueous solutions obtained were stabilized in a cryostat at temperatures between 0 to 25°C.

To obtain the final concentrations of three components (hyaluronic acid 1.0%, chitosan 1.8% and NaGP 18% by w/v, samples of known volumes of the 50% w/v aqueous solution of sodium NaGP and the 10% w/v aqueous solution of HA were added to a sample of known volume of the first aqueous solution of chitosan 4% (concentration of lactic acid = 0.6% w/v). The mixture of the three component aqueous solutions were mixed with vortex or magnetic stirring at room temperature, obtaining a homogeneous and clear solution with a final pH of 7.1. Example 6 *Preparation of a compositions of hyaluronic acid 1.0%, chitosan 3.0% and sodium glycerophosphate pentahydrate 18% by wlv.*

A first aqueous solution of chitosan (4% w/v) was prepared by adding to a water dispersion of chitosan, having a degree of deacetylation of 75%, lactic acid up to a concentration of 1 % w/v. Separately were prepared a 50% w/v aqueous solution of sodium glycerophosphate pentahydrate (NaGP) and a 10% w/v aqueous solution of sodium hyaluronate (HA). All the aqueous solutions were prepared at 0 °C. Then, the aqueous solutions obtained were stabilized in a cryostat at temperatures between 0 to 25°C.

To obtain the final concentrations of three components (hyaluronic acid 1.0%, chitosan 3.0% and NaGP 18% by w/v, samples of known volumes of the 50% w/v aqueous solution of NaGP and the 10% w/v aqueous solution of HA were added to a sample of known volume of the first aqueous solution of chitosan 4% (concentration of lactic acid = 0.9% w/v). The mixture of the three component aqueous solutions were mixed with vortex or magnetic stirring at room temperature, obtaining a homogeneous and clear solution with a final pH of 7.0.

Example 7 *Preparation of a compositions of hyaluronic acid 0.3%, chitosan 1.8% and sodium glycerophosphate penthaydrate 8.0% by wlv.*

A first aqueous solution of chitosan (4% w/v) was prepared by adding to a water dispersion of chitosan, having a degree of deacetylation of 75%, lactic acid up to a concentration of 1 % w/v. Separately were prepared a 50% w/v aqueous solution of sodium glycerophosphate pentahydrate (NaGP) and a 10% w/v aqueous solution of sodium hyaluronate (HA). All the aqueous solutions were prepared at 0 °C. Then, the aqueous solutions obtained were stabilized in a cryostat at temperatures between 0 to 25°C.

To obtain the final concentrations of three components (hyaluronic acid 0.3%, chitosan 1.8% and sodium NaGP 8% by w/v, samples of known volumes of the 50% w/v aqueous solution of NaGP and the 10% w/v aqueous solution of sodium HA were added to a sample of known volume of the first aqueous solution of chitosan 4% (concentration of lactic acid = 0.9% w/v). The mixture of the three component aqueous solutions were mixed with vortex or magnetic stirring at room temperature, obtaining a homogeneous and clear solution with a final pH of 6.8. Example 8 *Preparation of a compositions of hyaluronic acid 1.0%, chitosan 1.8% and sodium glycerophosphate pentahydrate 8.0% by wlv.*

The composition of example 2 was prepared with an alternative method which allows the instantaneous mixing of two distinct solutions.

The starting solutions were prepared at room temperature, were two times concentrated with respect to the final concentration in the desired composition and were mixed at the same rate through an automatic injector.

In more detail, a 3.6% w/v chitosan solution was prepared with 1.2% w/v lactic acid. In the meantime, a 32% w/v sodium glycerophosphate pentahydrate (NaGP) aqueous solution and a 4% w/v sodium hyaluronate (HA) aqueous solution were separately prepared then mixed in the same ratio, in order to have a 16% NaGP concentration and a 2% HA concentration.

5mL of each of the two resulting solution were picked up with 2 syringes which were mounted on an automatic injector set at the rate of 50µL/min. The two solutions were allowed to mix by linking the two syringes to a Y-connector.

In these conditions the resulting composition was a homogeneous and clear solution with a final pH of 7.1.

Example A *Preparation of a compositions of hyaluronic acid 0.3%, chitosan 3.0% and sodium glycerophosphate pentahydrate 8.0% by w/v.*

The preparation of example 1 was prepared using a chitosan having a degree of deacetylation of 95%. A phase separation with precipitation of coacervates was recorded.

Example B *Preparation of a compositions of hyaluronic acid 1.0%, chitosan 1.8% and sodium glycerophosphate pentahydrate 8.0% by w/v.*

The preparation of example 2 was prepared using a chitosan having a degree of deacetylation of 95%. A phase separation with precipitation of coacervates was recorded.

Example C *Preparation of a compositions of hyaluronic acid 1.0%, chitosan 3.0% and sodium glycerophosphate pentahydrate 8.0% by w/v.*

The preparation of example 3 was prepared using a chitosan having a degree of deacetylation of 95%. A phase separation with precipitation of coacervates was recorded.

Example D *Preparation of a compositions of hyaluronic acid 0.3%, chitosan 3.0% and sodium glycerophosphate pentahydrate 18% by w/v.*

The preparation of example 4 was prepared using a chitosan having a degree of deacetylation of 95%. A phase separation with precipitation of coacervates was recorded.

Example E *Preparation of a compositions of hyaluronic acid 1.0%, chitosan 1.8% and sodium glycerophosphate pentahydrate 18% by w/v.*

The preparation of example 5 was prepared using a chitosan having a degree of deacetylation of 95%. A phase separation with precipitation of coacervates was recorded.

Example F *Preparation of a compositions of hyaluronic acid 1.0%, chitosan 3.0% and sodium glycerophosphate pentahydrate 18% by w/v.*

The preparation of example 6 was prepared using a chitosan having a degree of deacetylation of 95%. A phase separation with precipitation of coacervates was recorded.

Example 9 *Turbidity evaluation of compositions of examples 1, 2 D and E*

The turbidity of the final solutions prepared according to examples 1, 2 D and E was determined by reading the transmittance of said solutions at 590 nm using a V530 UV-Vis spectrophotometer (Jasco, Japan) equipped with a quartz cuvette having an optical length of 1 cm.

The results are reported on table 1.

Table 1. Turbidity assay of the compositions of examples 1, 2 D and E

| Composition (Examples) | Transmittance of the solution % |
|---|---|
| 1 | 71.05 |
| 2 | 42.35 |
| D | 2.58 |

(continued)

| Composition (Examples) | Transmittance of the solution % |
| --- | --- |
| E | 4.05 |

Data reported in Table 1 indicate that the composition of Examples D and E were non-transparent solutions as they transmitted only 3-4 % of the incident light, while the composition of examples 1 and 2 were transparent and clear solutions. Example 10 *Thermogelation of the compositions prepared according to examples 1-6 in comparison with the compositions prepared according to examples A-F*

The sol-gel transition properties of the aqueous compositions prepared were evaluated by measuring the viscosity difference (Δ viscosity) between the viscosity measured at room temperature (15-25 °C) and after gelation at 37°C. The viscosity measurements were performed using a Brookfield type viscometer Visco-Star-R, equipped with a spindle type R7 (Fungila S.A., Italy).

The assay was carried out at the following conditions:

- starting temperature: 0 - 0.5 °C;
- stabilization time at the starting temperature: 15 minutes;
- stabilization time at room temperature (15-25°C): 30 minutes;
- heating in a water thermostatic bath at 37°C for 12-16 hours;
- spindle rate (100rpm).

The results are reported on table 2.

Table 2. Thermogelation assay of the compositions of example 1-6 and of comparison examples A-F

| Compositions (examples) | Chitosan (DD 75%) | HA | NaGP | Thermogelation (Δ viscosity) |
| --- | --- | --- | --- | --- |
| 1 | 3.0 | 0.3 | 8.0 | $3.75 \times 10^3$ |
| 2 | 1.8 | 1.0 | 8.0 | $1.7 \times 10^3$ |
| 3 | 3.0 | 1.0 | 8.0 | $1.95 \times 10^3$ |
| 4 | 3.0 | 0.3 | 18 | $4.65 \times 10^3$ |
| 5 | 1.8 | 1.0 | 18 | $1.85 \times 10^3$ |
| 6 | 3.0 | 1.0 | 18 | $4.10 \times 10^3$ |
| | | | | |
| Compositions (examples) | Chitosan (DD 95%) | HA | NaGP | Thermogelation (Δ viscosity) |
| A | 3.0 | 0.3 | 8.0 | - |
| B | 1.8 | 1.0 | 8.0 | - |
| C | 3.0 | 1.0 | 8.0 | - |
| D | 3.0 | 0.3 | 18.0 | - |
| E | 1.8 | 1.0 | 18.0 | - |
| F | 3.0 | 1.0 | 18.0 | - |

As shown by the results the degree of deacetylation of chitosan appeared to play a crucial role in sol-gel transition of the compositions tested.

A statistical analysis of the data reported in Table 2 evidences that, on thermogelation a high statistical significance of the effects was presented by the degree of deacetylation of the chitosan (p-Value = $3 \times 10^{-4}$). Lower statistical significance was presented by the effect of lactic acid concentration (p-Value = 0.025), whereas the effect of the concentration of chitosan, HA and NaGP was non-significant (p-Value = 0.15, 0.69 and 0.59 respectively).

This analysis of the effects allows to conclude that the concentration of HA and NaGP has practically no effect within the concentration range considered on the pH and the capability of the formulation to give rise to thermogelation, while the concentration of lactic acid proved to be a critical parameter both for the pH and the thermogelation.

The pH was unaffected by the heating treatment for the thermogelation.

Example 11 *Thermogelation of the compositions prepared according to examples 1, 3, 4 and 6 at different temperatures*

The compositions were tested in order to find out the minimum temperature at which the thermogelation takes place. Four different temperature were considered, namely 30, 34.5, 35.5 and 37.5 °C.

The obtained results are reported in Table 3.

Table 3. Evaluation of the viscosity increase upon heating at different temperatures

| Compositions (examples) | 30±0.5°C | 34.5±0.5°C | 35.5±0.5°C | 37.5±0.5°C |
|---|---|---|---|---|
| 1 | No gelation | No gelation | No gelation | High gelation |
| 3 | No gelation | No gelation | No gelation | High gelation |
| 4 | Only opalescence | High gelation | High gelation | High gelation |
| 6 | No gelation | High gelation | High gelation | High gelation |

It can be observed that none of the compositions tested gave rise to thermogelation at 30 °C. The compositions of examples 4 and 6 underwent sol-gel transition at 34.5 °C whereas for the compositions of examples 1 and 3 the phenomenon was observed around 37 °C.

Example 12 *Differential scanning calorimetry (DSC) analysis of the compositions prepared according to examples 1, 2, 3, 7 and 8*

DSC analysis used to measure the amount of free (freezable) water was performed with a DSC 821 e (Mettler Toledo, USA) driven by a STARe software (Mettler Toledo, USA).This determination is based on the measurement of the enthalpy of fusion of the water in the sample in comparison with the enthalpy of fusion of pure water per unit weight according to the following equation (1):

$$F_W = \Delta Hf_s/(\Delta Hf_{pw}/m_{pw})$$

$$(1)$$

where:

$F_w$ is the mass of free water in the tested sample; $\Delta Hf_s$ is the enthalpy of fusion of the water in the tested sample; $\Delta Hf_{pw}$ is the enthalpy of fusion of a sample of pure water with mass $m_{pw}$.

The percentage of bound water, $B_w$, was calculated according to the following equation (2):

$$B_W = (T_W - F_W)/T_W \times 100$$

$$(2)$$

where:

$Tw$ is the total amount of water in the sample as determined by TGA (TG 50, Mettler Toledo USA, driven by STARe software, Mettler Toledo Switzerland).

The percentage of the bound water in the samples is reported in Table 4 with relevant ratio between chitosan and hyaluronic acid.

Table 4. Ratio between concentration (%) of chitosan and concentration of HA for the different formulations and the relevant percentage of bound water.

| Compositions (examples). | chitosan/HA ratio | % bound water |
|---|---|---|
| 2 | 1.8 | 8.09 |

(continued)

| Compositions (examples). | chitosan/HA ratio | % bound water |
|---|---|---|
| 3 | 3 | 9.07 |
| 7 | 6 | 10.57 |
| 1 | 10 | 12.77 |

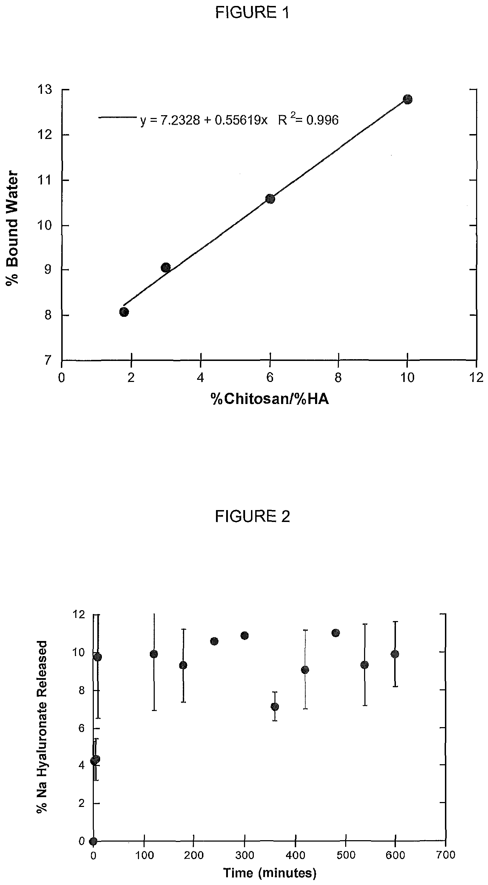

A linear correlation ($R^2$ = 0.996) was found between concentration ratio chitosan/hyaluronic acid and the percentage of bound water (Figure 1).

This indicates that as the ratio between chitosan and HA increases the percentage of bound water increases as well. This could be related to the interaction between chitosan and HA rather than simply to the better capability of chitosan to interact with water with respect to HA. In fact, chitosan is more available for binding water in those compositions in which it is less involved in interactions with hyaluronic acid.

A composition prepared according to example 8 having half the concentration of chitosan (0.9%) was also tested. This formulation was not completely clear suggesting incomplete hydration of the components. Moreover, it failed in thermogelation. DSC and TGA analyses indicated absence of bound water in the starting solution.

Example 13 *Mucoadhesiveness assay of the compositions prepared according to examples 1 and 2: ex vivo mucoadhesion*

The mucoadhesive potential of the compositions was determined by wash-off tests adapted from the method reported by Lehr et al. (Lehr A. et al., Int. J. Pharmaceutics, 1992, 78, 43-48).

Freshly excised pig (Large White, Landrance, 10-11 months old, 145-190 kg weight) intestines were obtained from a local slaughterhouse (Parma, Italy) within an hour of slaughter. Intestine explant (at least 30 cm of descending colon, cut from the left flexure and the sigma) was carried out by a veterinary. The freshly excised pig intestine was transported to the laboratory in isotonic saline solution in an ice bath within one hour from the sacrifice. After extensive washing with isotonic saline solution (500 mL for at least three times), each intestine specimen was cut along its length and the mucosa was carefully separated from the underlying connective tissue with surgical scissors paying attention not to damage the mucosal structure.

A piece of pig intestinal tissue (about 2 cm x 4 cm) was pasted onto a glass slide using cyanoacrylate glue, keeping the luminal side face up. A given weight of composition was spread uniformly over the intestinal mucosal tissue then the glass slides were kept for 15 minutes in a 37°C oven. Each glass slide was then hung on the arm of a Ph. Eur. 7.0 tablet disintegrating apparatus (Erweka, Germany)and regularly (30 strokes/min) dipped in 30 mL of phosphate buffer solution (pH 6.8) at 37±0.5 °C by operating the Ph. Eur. 7.0 tablet disintegrating test apparatus.

At given time intervals 1 mL of solution was collected and replaced by fresh buffer. Collected samples were frozen before further treatment for quantification. Samples drawn from the wash-off test were then treated and analyzed by HPLC-ESI-MS for the quantification of HA released from the composition into the buffer solution. The HA release was taken as a negative indication of the capability of the composition to keep in contact with the mucosa over time.

Compositions prepared according to examples 1 and 2 were tested. Two compositions of Carbopol® gel containing the same percentage of HA were used for control reference.

Preparation of samples for analysis

Hyaluronic acid is a naturally occurring high molecular weight linear polysaccharide. The repeating disaccharide units consist of glucuronic acid and N-acetylglucosamine via a β-1,4 linkage. Direct quantification of HA in solution is not feasible for a large number of samples such as that obtained from these release experiments. HA is usually quantified by quantification of one or more HA degradation products. Since enzymatic degradation of HA is expensive and time consuming, methods including alkaline or acid degradation are preferred. In this study, an acid degradation protocol was utilized. Adopting the method reported by Alkrad et al (AlkradJ.A. et al., J. Pharmaceutical and Biomedical Analysis, 2002, 30, 913-919): each sample (1 mL) obtained from the wash-off experiment was dried out then treated with 250 μL of a 47.5% (v/v) solution of sulfuric acid. Samples were kept at 45 °C for 10 minutes then the reaction was stopped by neutralizing with sodium hydroxide solutions to reach pH 5. Each sample was brought to a final volume of 3 mL with distilled water then filtered through a 0.45 μm cellulose acetate filter and kept at -20°C before LC-MS analysis.

Liquid chromatography-electrospray-mass spectrometry method

HPLC separation was performed on a 5 $\mu$m Atlantis® dC18 100 A column (2.1 mm x 100 mm) (Waters Corporation, USA). The mobile phase was delivered by a binary pump (Agilent 1100 series) and elution was performed under gradient mode by using a formic acid aqueous solution (pH 3.5) (eluent A) and methanol (eluent B) at a flow rate of 0.2 mL/min. The sample ($V_{inj}$: 20 $\mu$L) was injected in a gradient set as follows:

| Time (min) | A (%) | B (%) |
|---|---|---|
| 1 | 98 | 2 |
| 2 | 98 | 2 |
| 3 | 60 | 40 |
| 4 | 60 | 40 |
| 5 | 20 | 80 |
| 8 | 20 | 80 |
| 9 | 98 | 2 |
| 13 | 98 | 2 |

ESI mass spectra were recorded using an MS API 150EX equipped with a Turboion spray source (Applied Biosystems, USA). The electrospray interface parameters were set as follows: capillary voltage 4.5 kV, source temperature 450 °C, de-clustering potential 50V, focusing potential 400V. For quantitative analysis signals were acquired in positive ionization under SIM mode by monitoring ions at m/z 380, m/z 759. Nitrogen was used as a curtain (10 L/hour) and nebulizing gas (10 L/hour).

Results of the mucoadhesiveness study

A. HA release in the wash-off experiments

HA was mixed with chitosan and Carbopol®-based compositions. By performing wash-off experiments as described above, the release of HA from composition of examples 1 and 2 laid on pig mucosa in PBS solution kept at 37 °C was monitored as a function of time.

The composition prepared according to example 1 (containing 0.3% HA) released about 10% of the initial amount of HA in the first 10 minutes, thereafter the HA concentration in the buffer solution leveled off indicating no more HA release from the composition (Figure 2). The initial burst is likely ascribable to the mechanical drop of the upper layer of the formulation due to gravity after the first strokes of the test.

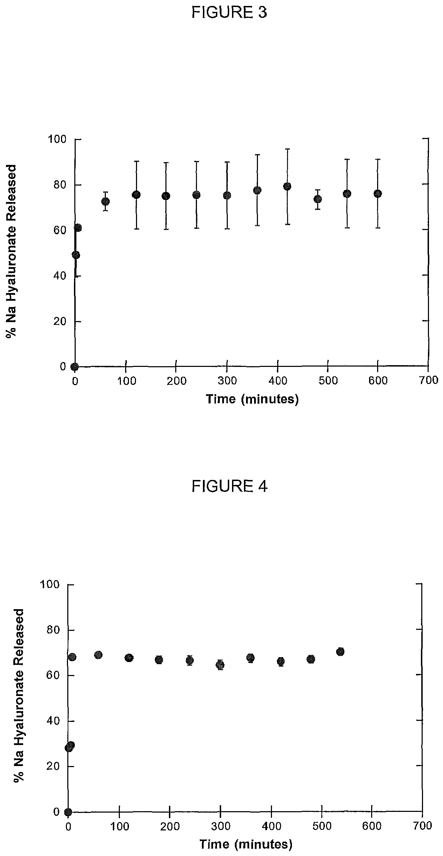

The corresponding Carbopol®-based composition showed similar release profile but shifted to higher % HA values. In fact, the amount of HA released after 10 minutes was approximately 55% of the dose. Also in this case a plateau was observed along the remaining part of the experiment (Figure 3).

In the case of the chitosan-based composition prepared according to example 2 (containing 1% HA) a release of about 60% of the HA dose was monitored in the first 45 minutes followed by a very low increase in the HA concentration that reached 70% after 10 hours (Figure 4).

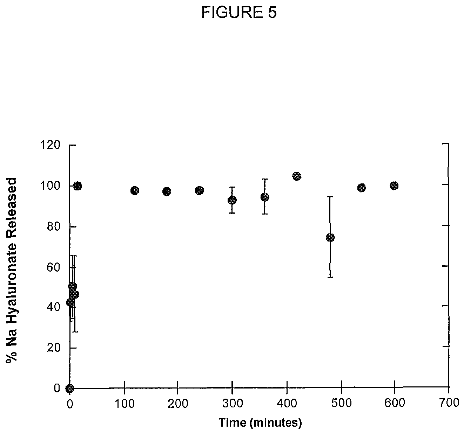

In the case of the Carbopol®-based composition corresponding to composition 2 the HA release was complete in less than 45 minutes (Figure 5).

The results clearly indicated the higher mucoadhesiveness of the chitosan-based-composition both in the cases of compositions prepared according to examples 1 and 2 with respect to the corresponding Carbopol®-based compositions.

**Claims**

1. A composition comprising hyaluronic acid and salts thereof in admixture with chitosan and salts thereof and a salt of glycerophosphate in an aqueous solution, wherein the chitosan has a deacetylation degree in the range from 60 to 80% and said composition has a sol-gel transition property at 35-38 °C so forming at said temperatures a hydrogel.

2. The composition according to claim 1, wherein the ratios between hyaluronic acid and chitosan are from 1:0.6 to 1:45 (HA:chitosan).

3. The composition according to claim 1, wherein the ratios between chitosan and glycerophosphate are from 1:0.8 to 1:15 (chitosan:glycerophosphate).

4. The composition according to claim 2, wherein the ratios between hyaluronic acid and chitosan are from 1:10 to 1:1.8 (HA:chitosan).

5. The composition according to claim 3, wherein the ratios between chitosan and glycerophosphate are from 1:1.8 to 1:7.0 (chitosan:glycerophosphate).

6. The composition according to claim 1, wherein the contents of the components are comprised in the ranges:

   - hyaluronic acid from 0.1 to 1.5 % w/v;
   - chitosan from 1.0 to 4.5 % w/v; and
   - anhydrous glycerophosphate from 4.0 to 15.0 % w/, and

   wherein the pH is comprised from 6.9 to 7.1.

7. The composition according to claim 6, wherein the contents of the components are comprised in the ranges:

   - hyaluronic acid from 0.3 to 1.0 % w/v;
   - chitosan from 1.8 to 3.0 % w/v; and
   - anhydrous glycerophosphate from 5.6 to 12.7 % w/v.

8. The composition according to claim 7, wherein the glycerophospate is 5.6 % by w/v.

9. The composition according to claim 1, wherein the hyaluronic acid has a molecular weight comprised from 7 to 250 kDa.

10. The composition according to claim 9, wherein the hyaluronic acid has a molecular weight comprised from 30 to 250 kDa.

11. The composition according to claim 1, wherein the chitosan has a deacetylation degree of 70-80%.

12. The composition according to one of the claims 1-11, wherein the glycerophosphate is in form of hydrate crystalline with 4 to 6 molecules of water of crystallization.

13. The composition according to one of the claims 1-12, further comprising an inorganic or organic acid selected from hydrochloric, sulfuric, acetic, lactic sulfuric ascorbic, malic, succinic, adipic or fumaric acid in a range from 0.6 to 0.9 % w/v.

14. The composition according to one of the claims from 1 to 13 for use in promoting the wound healing of mucosa of the body cavities by topical administration.

15. The composition for use according to claim 14, wherein the use is for wound healing of oral, nasal, gastrointestinal, colorectal and vaginal mucosa.

16. The composition for use according to claim 14, wherein the use is for wound healing of mucosal lesions derived from chemotherapy and/or radiotherapy used to treat cancer.


**Patentansprüche**

1. Mischung, die Hyaluronsäure und Salze davon aufweist, mit Beigabe von Chitosan und Salzen davon und einem Salz von Glyzerophosphat in wässriger Lösung, wobei das Chitosan einen Deacetylierungsgrad zwischen 60 und 80 % und die Mischung einen Sol-Gel-Übergang bei 35 -38 °C hat, so dass bei diesen Temperaturen ein Hydrogel gebildet wird.

2. Mischung gemäß Anspruch 1, wobei das Verhältnis von Hyaluronsäure zu Chitosan im Bereich zwischen 1 : 0,6 und 1 : 45 (HS : Chitosan) liegt.

3. Mischung gemäß Anspruch 1, wobei das Verhältnis von Chitosan zu Glyzerophosphat im Bereich zwischen 1 : 0,8 und 1 : 15 (Chitosan : Glyzerophosphat) liegt.

4. Mischung gemäß Anspruch 2, wobei das Verhältnis von Hyaluronsäure zu Chitosan im Bereich zwischen 1 : 10 und 1 : 1,8 (HS : Chitosan) liegt.

5. Mischung gemäß Anspruch 3, wobei das Verhältnis von Chitosan zu Glyzerophosphat im Bereich zwischen 1 : 1,8 und 1 : 7,0 (Chitosan : Glyzerophosphat) liegt.

6. Mischung gemäß Anspruch 1, wobei die Mengen der Komponenten in folgenden Bereichen vorliegen:

   - Hyaluronsäure von 0,1 bis 1,5 % Volumengewicht
   - Chitosan von 1,0 bis 4,5 % Volumengewicht, und
   - anhydrisches Glyzerophosphat von 4,0 bis 15,0 % Volumengewicht, wobei der pH-Wert zwischen 6,9 und 7,1 liegt.

7. Mischung gemäß Anspruch 6, wobei die Mengen der Komponenten in folgenden Bereichen vorliegen:

   - Hyaluronsäure von 0,3 bis 1,0 % Volumengewicht
   - Chitosan von 1,8 bis 3,0 % Volumengewicht, und
   - anhydrisches Glyzerophosphat von 5,6 bis 12,7 % Volumengewicht.

8. Mischung gemäß Anspruch 7, wobei das Glyzerophosphat mit 5,6 % Volumengewicht vorliegt.

9. Mischung gemäß Anspruch 1, wobei die Hyaluronsäure ein Molekulargewicht zwischen 7 und 250 kDa aufweist.

10. Mischung gemäß Anspruch 9, wobei die Hyaluronsäure ein Molekulargewicht zwischen 30 und 250 kDa aufweist.

11. Mischung gemäß Anspruch 1, wobei das Chitosan einen Deacetylierungsgrad zwischen 70 und 80 % aufweist.

12. Mischung gemäß einem der Ansprüche 1 bis 11, wobei das Glyzerophosphat in Form von Hydratkristallen mit 4 bis 6 Molekülen Kristallwasser vorliegt.

13. Mischung gemäß einem der Ansprüche 1 bis 12, die zudem im Bereich von 0,6 bis 0,9 % Volumengewicht eine anorganische oder organische Säure aus der Gruppe von Salzsäure, Schwefelsäure, Essigsäure, milchig schwefeliger Ascorbinsäure, Apfelsäure, Bernsteinsäure, Adipinsäure oder Fumarsäure aufweist.

14. Mischung gemäß einem der Ansprüche 1 bis 13 zur Verwendung zur Förderung der Wundheilung der Schleimhäute von Körperöffnungen bei topischer Behandlung.

15. Mischung zur Verwendung gemäß Anspruch 14, wobei die Verwendung zur Wundheilung von oralen, nasalen, gastrointestinalen, kolorektalen und vaginalen Schleimhäuten dient.

16. Mischung zur Verwendung gemäß Anspruch 14, wobei die Verwendung zur Wundheilung von Schleimhautverletzungen dient, die von Chemotherapien und / oder Strahlentherapien bei der Krebsbehandlung stammen.


**Revendications**

1. Composition comprenant de l'acide hyaluronique et ses sels en mélange avec du chitosane et ses sels et un sel de glycérophosphate dans une solution aqueuse, dans laquelle le chitosane présente un degré de désacétylation situé dans la plage de 60 à 80 % et ladite composition est dotée d'une propriété de transition sol-gel à une température de 35 à 38 °C formant ainsi auxdites températures un hydrogel.

**2.** Composition selon la revendication 1, dans laquelle les rapports entre l'acide hyaluronique et le chitosane sont de 1/0,6 à 1/45 (HA/chitosane).

**3.** Composition selon la revendication 1, dans laquelle les rapports entre le chitosane et le glycérophosphate sont de 1/0,8 à 1/15 (chitosane/glycérophosphate).

**4.** Composition selon la revendication 2, dans laquelle les rapports entre l'acide hyaluronique et le chitosane sont de 1/10 à 1/1,8 (HA/chitosane).

**5.** Composition selon la revendication 3, dans laquelle les rapports entre le chitosane et le glycérophosphate sont de 1/1,8 à 1/7,0 (chitosane/glycérophosphate).

**6.** Composition selon la revendication 1, dans laquelle les teneurs des composants sont comprises dans les plages :

- acide hyaluronique de 0,1 à 1,5 % p/v ;
- chitosane de 1,0 à 4,5 % p/v ; et
- glycérophosphate anhydre de 4,0 à 15,0 % p/v, et dans laquelle le pH est compris de 6,9 à 7,1.

**7.** Composition selon la revendication 6, dans laquelle les teneurs des composants sont comprises dans les plages :

- acide hyaluronique de 0,3 à 1,0 % p/v ;
- chitosane de 1,8 à 3,0 % p/v ; et
- glycérophosphate anhydre de 5,6 à 12,7 % p/v.

**8.** Composition selon la revendication 7, dans laquelle le glycérophosphate est de 5,6 % en p/v.

**9.** Composition selon la revendication 1, dans laquelle l'acide hyaluronique a un poids moléculaire compris de 7 à 250 kDa.

**10.** Composition selon la revendication 9, dans laquelle l'acide hyaluronique a un poids moléculaire compris de 30 à 250 kDa.

**11.** Composition selon la revendication 1, dans laquelle le chitosane présente un degré de désacétylation de 70 à 80 %.

**12.** Composition selon l'une des revendications 1 à 11, dans laquelle le glycérophosphate est sous la forme d'un hydrate cristallisé avec 4 à 6 molécules d'eau de cristallisation.

**13.** Composition selon l'une des revendications 1 à 12, comprenant en outre un acide inorganique ou organique choisi parmi les acides chlorhydrique, sulfurique, acétique, lactique sulfurique ascorbique, malique, succinique, adipique ou fumarique dans une plage de 0,6 à 0,9 % p/v.

**14.** Composition selon l'une des revendications 1 à 13 pour une utilisation dans l'activation de la cicatrisation des muqueuses des cavités corporelles par administration topique.

**15.** Composition pour une utilisation selon la revendication 14, l'utilisation étant pour la cicatrisation des muqueuses orale, nasale, gastro-intestinale, colorectale et vaginale.

**16.** Composition pour une utilisation selon la revendication 14, l'utilisation étant pour la cicatrisation de lésions des muqueuses dérivées d'une chimiothérapie et/ou d'une radiothérapie utilisée pour traiter le cancer.

FIGURE 1

$y = 7.2328 + 0.55619x$  $R^2 = 0.996$

FIGURE 2

FIGURE 3

FIGURE 4

FIGURE 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5925626 A
- WO 2009024677 A
- WO 9603973 A
- US 6344488 B
- US 20060280797 A

### Non-patent literature cited in the description

- **BERGER J. et al.** *Eur. J. Pharmaceutics and Biopharmaceutics,* 2004, vol. 57, 35-52
- **CHENITE A. et al.** *Biomaterials,* 2000, vol. 21, 2155-2161
- **CHENITE A. et al.** *Carbohydr. Polym.,* 2001, vol. 46, 39-47
- **RUEL-GARIÉPY E. et al.** *Int. J. Pharmaceutics,* 2000, vol. 203, 89-98
- **FANG et al.** *Eur. J. Pharmaceutics and Biopharmaceutics,* 2008, vol. 68, 626-636
- **CHEN et al.** *Polymer,* 2009, vol. 50, 107-116
- **LEHR A. et al.** *Int. J. Pharmaceutics,* 1992, vol. 78, 43-48
- **ALKRADJ.A. et al.** *J. Pharmaceutical and Biomedical Analysis,* 2002, vol. 30, 913-919